# EUROPÄISCHE PATENTANMELDUNG

(11) **EP 4 548 948 A1**
(43) Veröffentlichungstag der Anmeldung: **07.05.2025**
(21) Anmeldenummer: 23207091.2
(22) Anmeldetag: 31.10.2023
(51) Int. Cl.: A61M 5/20, A61M 5/32

(54) **VORRICHTUNG ZUM VERABREICHEN EINER FLUIDEN SUBSTANZ**

(71) Anmelder: Ypsomed AG, 3401 Burgdorf (CH)
(72) Erfinder: Loser, Peter, 3037 Herrenschwanden (CH); Tschirren, Markus, 3400 Burgdorf (CH); Schrul, Christian, 3400 Burgdorf (CH); Glauser, Oliver, 3008 Bern (CH); Zumstein, Dominik, 3014 Bern (CH); Bosshard, Simon Martin, 3324 Hindelbank (CH)
(74) Vertreter: Eugster, Monika Katharina

(57) **Zusammenfassung**

Die Erfindung betrifft eine Vorrichtung und ein Verfahren zum Montieren dieser Vorrichtung zum Verabreichen einer fluiden Substanz. Die erfindungsgemässe Vorrichtung umfasst ein Gehäuse (1) und ein in dem Gehäuse (1) axial fest aufgenommenen Produktbehälter (2) mit einer Nadel (2a), wobei die Substanz durch die Nadel (2a) verabreichbar ist. Zudem umfasst die erfindungsgemässe Vorrichtung eine an dem Gehäuse (1) vorgesehene nach innen gerichtete Schulterauflage (1a), an welcher sich der Produktbehälter (2) mit einer distal angeordneten Schulter (2b) abstützt, um eine axiale relative Bewegung zwischen dem Produktbehälter (2) und dem Gehäuse (1) in die distale Richtung zu verhindern.

## Beschreibung

Die Erfindung betrifft eine Vorrichtung mit einer Längsachse (L) und ein Montieren dieser Vorrichtung zum Verabreichen einer fluiden Substanz, insbesondere eines Medikaments.

Der Begriff «Medikament» umfasst hier jede fliessfähige medizinische Formulierung, welche geeignet ist zur kontrollierten Verabreichung durch ein Mittel wie eine Kanüle oder Nadel hindurch, beispielsweise umfassend eine Flüssigkeit, eine Lösung, ein Gel, oder eine feine Suspension, welche einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzymen und weitere Substanzen sowohl in fester (suspendierter) oder flüssiger Form aber auch Polysaccaride, Vaccine, DNS oder RNS oder Oglionukletoide, Antikörper oder Teile von Antikörpern sowie geeignete Basis-, Hilfs- und Trägerstoffe.

Aus dem Stand der Technik sind Vorrichtungen zum Verabreichen einer fluiden Substanz bekannt. Aus der EP2742962B1 ist beispielsweise ein Autoinjektor mit einem Gehäuse und einer Spritze bekannt, wobei die Spritze axial fest in einem Spritzenhalter aufgenommen ist. Der Spritzenhalter weist eine Schulterauflage auf, die dazu dient, die Spritze an einer Schulter gegen eine Bewegung entlang der Längsachse (L) in die distale Richtung relativ zu dem Spritzenhalter zu sichern. Zudem weist das Gehäuse einen Halteabschnitt auf, welcher die Schulterauflage des Spritzenhalters in Eingriff mit der Schulter der Spritze hält, um die Spritze in dem Autoinjektor gegen eine Bewegung entlang der Längsachse (L) in die distale Richtung zu sichern.

Nachteilig bei diesem Autoinjektor ist, dass dieser Autoinjektor komplex aufgebaut ist.

Es ist eine Aufgabe der Erfindung, eine alternative Vorrichtung und ein Montieren dieser Vorrichtung zum Verabreichen einer fluiden Substanz bereitzustellen, welche einfach und sicher aufgebaut ist.

Im Folgenden bedeutet die proximale Richtung bei einer Vorrichtung zum Verabreichen einer fluiden Substanz die Richtung zum gehäuseseitigen Ende hin und die distale Richtung bei dieser Vorrichtung die Richtung zum nadelseitigen Ende hin.

Die erfindungsgemässe Vorrichtung mit einer Längsachse (L) zum Verabreichen einer fluiden Substanz, insbesondere eines Medikaments weist ein Gehäuse auf. Das Gehäuse ist bevorzugt länglich ausgebildet. Das Gehäuse kann bevorzugt hülsenförmig und/oder zylindrisch, insbesondere kreiszylindrisch ausgebildet sein. In dem Gehäuse ist ein Produktbehälter axial fest aufgenommen. Der Produktbehälter umfasst die zu verabreichende Substanz. Der Produktbehälter weist eine Nadel auf, durch welche die Substanz verabreicht werden kann. Der Produktbehälter kann als Spritze, welche einen Spritzenkörper und eine an einem distalen Ende des Spritzenkörpers aufgenommene Nadel umfasst, ausgebildet sein. Alternativ kann der Produktbehälter als Karpule mit einem Karpulenkörper und einer aufsetzbaren und entfernbaren Nadeleinheit ausgebildet sein. Die Nadel oder die Nadeleinheit kann eine entfernbare Nadelschutzhülse aufweisen, um die Nadel vor Verschmutzungen zu schützen und um die Nadel und die zu verabreichende Substanz steril zu halten.

Die erfindungsgemässe Vorrichtung umfasst ferner eine an dem Gehäuse vorgesehene nach innen gerichtete Schulterauflage, an welcher sich der Produktbehälter mit einer distal angeordneten Schulter abstützt, um eine axiale relative Bewegung zwischen dem Produktbehälter und dem Gehäuse in die distale Richtung zu verhindern. Die Schulter des Produktbehälters ist als sich verjüngenden Abschnitt im distalen Bereich des Produktbehälters, insbesondere im distalen Bereich des Spritzenkörpers oder des Karpulenkörpers ausgebildet. Die nach innen gerichtete Schulterauflage des Gehäuses ist bevorzugt elastisch, insbesondere als elastischer Haltearm oder elastischer Schnapparm ausgebildet. Die Schulterauflage ist derart ausgebildet, dass sie in Eingriff mit der Schulter des Produktbehälters gelangen kann, um eine axiale relative Bewegung zwischen dem Produktbehälter und dem Gehäuse in die distale Richtung zu verhindern. Die Schulterauflage des Gehäuses ist radial nach aussen vorspannbar oder radial auslenkbar ausgebildet. Die Nadel des Produktbehälters ist mit der entfernbaren Nadelschutzhülse abgedeckt, wobei die nach innen gerichtete Schulterauflage des Gehäuses zwischen einem proximalen Ende der Nadelschutzhülse und der distalen Schulter des Produktbehälters eingeschnappt ist oder eingreift, wenn der Produktbehälter in dem Gehäuse axial fest aufgenommen ist. Im Eingriff ist die Schulterauflage entspannt oder eingeschnappt, wobei die Schulter des Produktbehälters an dem Schulterauflage des Gehäuses abgestützt ist. Die Schulterauflage des Gehäuses kann bevorzugt einteilig oder einstückig mit dem Gehäuse ausgebildet sein. Dadurch kann erzielt werden, dass die Vorrichtung mit wenigen Bestandteilen und kostengünstig aufgebaut ist, da bei der Montage der Vorrichtung auf die Verwendung des Produktbehälterhalters zum Halten und Einfügen des Produktbehälters in das Gehäuse der Vorrichtung verzichtet werden kann. Alternativ kann die Schulterauflage und das Gehäuse zweitteilig oder zweistückig ausgebildet sein, wobei die Schulterauflage axial- und drehfest zu dem Gehäuse angeordnet ist. Dadurch kann das Montieren der Vorrichtung vereinfacht werden.

Des Weiteren umfasst die erfindungsgemässe Vorrichtung ein relativ zu dem Gehäuse verschiebbares Nadelschutzelement für die Nadel. Das Nadelschutzelement kann hülsenförmig und/oder zylindrisch, insbesondere kreiszylindrisch ausgebildet sein. Das Nadelschutzelement kann zum Schutz vor Stichverletzungen durch die Nadel, insbesondere durch eine distale Spitze der Nadel dienen. Ferner weist die Vorrichtung eine Nadelschutzfeder auf, welche mit dem Nadelschutzelement gekoppelt ist, wobei das Nadelschutzelement von einer distalen Position, in welcher die distale Spitze der Nadel von dem Nadelschutzelement umgeben ist und in eine proximale Position, in welcher die distale Spitze der Nadel von dem Nadelschutzelement freigeben ist, axial bewegbar ist.

Ferner weist die erfindungsgemässe Vorrichtung eine Antriebseinrichtung, welche durch eine Ausschüttfeder in eine Ausschüttposition getrieben wird, um die Substanz aus dem Produktbehälter zu verabreichen. Die Ausschüttfeder stellt die Energie zur Bewirkung der Substanzverabreichung zur Verfügung, um die Substanz aus dem Produktbehälter durch die Nadel zu verabreichen. Die Vorrichtung kann als Autoinjektor ausgebildet sein. Autoinjektoren werden oft als Vorrichtungen bezeichnet, welche eine zu verabreichende Substanz nach der Auslösung automatisch einem Patienten abgeben. Bevorzugt kann durch die axiale Bewegung des Nadelschutzelements von der distalen Position in die proximale Position die Antriebseinrichtung gelöst werden, um die Substanz aus dem Produktbehälter zu verabreichen. Alternativ kann die Auslösung der Vorrichtung durch eine Betätigung eines Auslöseknopfs erreicht werden. Die Vorrichtung kann derart ausgebildet sein, dass ein manuelles oder automatisches Einstechen der Nadel in die Haut des Patienten und/oder ein manuelles oder automatisches Zurückziehen der Nadel in das Gehäuse der Vorrichtung vorgesehen ist.

Die Nadelschutzfeder und die Ausschüttfeder können als Druckfeder ausgebildet sein. Besonders bevorzugt sind die beiden Federn als Wendelfeder ausgebildet. Es können aber auch andere Federn vorgesehen sein. Die Nadelschutzfeder und die Ausschüttfeder sind spannbar und entspannbar ausgebildet. Die Kraft der Nadelschutzfeder bewirkt, dass die Nadelschutzfeder das Nadelschutzelement von der distalen Position in die proximale Position und wieder in die distale Position oder in eine von der distalen Position unterschiedliche distale Position verschoben werden können. Die Kraft der Ausschüttfeder bewirkt, dass die Ausschüttfeder die gesamte Menge der zu verabreichenden Substanz oder eine Teilmenge der zu verabreichenden Substanz ausschütten kann. Die Nadelschutzfeder und die Ausschüttfeder können vorzugsweise aus Metall gefertigt sein. Es können auch andere Materialien, wie beispielsweise Kunststoff vorgesehen sein.

Die erfindungsgemässe Vorrichtung umfasst des Weiteren eine an dem Gehäuse vorgesehene Ausgleichsfeder, welche sich an einem proximalen Ende des Produktbehälters abstützt und über die sich der Produktbehälter in und gegen die Ausschüttrichtung elastisch an dem Gehäuse abstützt. Die Ausgleichsfeder kann einteilig oder einstückig mit dem Gehäuse ausgebildet sein. Dadurch kann erzielt werden, dass die Vorrichtung mit wenigen Bestandteilen und kostengünstig aufgebaut ist, da bei der Montage der Vorrichtung auf die Verwendung der Ausgleichsfeder der Vorrichtung verzichtet werden kann. Alternativ kann die Ausgleichsfeder und das Gehäuse zweiteilig oder zweistückig ausgebildet sein, wobei die Ausgleichsfeder und das Gehäuse axial- und drehfest zueinander angeordnet sind. Dadurch kann erzielt werden, dass die Vorrichtung einfach montiert werden kann. Die Ausgleichsfeder ist spannbar und entspannbar ausgebildet. Die Ausgleichsfeder ist derart ausgebildet, dass sich die Ausgleichsfeder an dem proximalen Ende des Produktbehälters abstützt und, dass über die Ausgleichsfeder sich der Produktbehälter in und gegen die Ausschüttrichtung elastisch an dem Gehäuse abstützt. Dadurch können Längenunterschiede des Produktbehälters ausgeglichen werden, die aufgrund von Herstellungstoleranzen entstehen können. Ferner kann der feste Sitz des Produktbehälters an der Schulterauflage des Gehäuses sichergestellt werden. Die Ausgleichsfeder ist vorzugsweise aus Kunststoff gefertigt. Es können auch andere Materialien, wie beispielsweise Metall vorgesehen sein.

Das Nadelschutzelement kann in der distalen und/oder in der proximalen Position ein radiales nach aussen Vorspannen oder ein radiales Auslenken der Schulterauflage des Gehäuses verhindern. Dadurch kann bei dem in dem Gehäuse axial fest aufgenommenen Produktbehälter eine axiale relative Bewegung zwischen dem Produktbehälter und dem Gehäuse in die distale Richtung verhindert werden. Die Nadelschutzhülse hält die Schulterauflage des Gehäuses in Eingriff mit der Schulter des Produktbehälters, um den Produktbehälter in der Vorrichtung gegen eine Bewegung entlang der Längsachse (L) in die distale Richtung zu sichern.

Die Erfindung betrifft des Weiteren ein Verfahren zum Montieren dieser erfindungsgemässen Vorrichtung. In einem ersten Schritt wird ein Gehäuse mit einer nach innen gerichteten Schulterauflage zur Aufnahme eines Produktbehälters bereitgestellt. Ferner wird ein Produktbehälter mit einer distal angeordneten Schulter und einer Nadel, welche mit einer entfernbaren Nadelschutzhülse abgedeckt ist, bereitgestellt. In einem weiteren Schritt wird der Produktbehälter in das Gehäuse entlang einer Längsachse (L) in eine distale Richtung verschoben oder eingesetzt. Bei dieser axialen relativen Bewegung zwischen dem Produktbehälters und dem Gehäuse, insbesondere bei dieser Verschiebung oder bei diesem Einsetzen des Produktbehälters in das Gehäuse gleitet die nach innen gerichtete Schulterauflage des Gehäuses über eine äussere Oberfläche der Nadelschutzhülse und wird dadurch radial nach aussen vorgespannt oder radial ausgelenkt. Wenn die radial nach aussen vorgespannte oder die radial ausgelenkte Schulterauflage des Gehäuses zwischen einem proximalen Ende der Nadelschutzhülse und der distal angeordneten Schulter des Produktbehälters gelangt oder positioniert ist, kann sich die radial nach aussen vorgespannt oder die radial ausgelenkte Schulterauflage zwischen dem proximalen Ende der Nadelschutzhülse und der distal angeordneten Schulter entspannen oder einschnappen. Danach kann das Nadelschutzelement relativ zu dem Gehäuse und in das Gehäuse in die proximale Richtung bis zu einer Stoppposition der Vorrichtung verschoben werden, bei der ein erneutes radiales nach aussen Vorspannen oder ein erneutes radiales Auslenken der Schulterauflage des Gehäuses verhindert wird. Der Vorteil dieses Verfahrens ist, dass wenige Montageschritte verwendet werden müssen, um die erfindungsgemässe Vorrichtung zu erstellen. Bei der Montage kann auf das Bereitstellen eines Produktbehälterhalters zum Halten und Einfügen des Produktbehälters in das Gehäuse der Vorrichtung verzichtet werden kann. Dadurch kann eine kostengünstige erfindungsgemässe Vorrichtung bereitgestellt werden.

Ferner kann das Gehäuse der erfindungsgemässen Vorrichtung einen Gehäuseanschlag und das Nadelschutzelement einen Nadelschutzanschlag aufweisen, welche die Stoppposition des Verschiebens der Nadelschutzelements zu dem Gehäuse und in das Gehäuse definieren, bei der ein erneutes radiales nach aussen Vorspannen oder ein erneutes radiales Auslenken der Schulterauflage verhindert wird. Der Gehäuseanschlag und/oder der Nadelschutzanschlag kann elastisch ausgebildet sein. In der Stoppposition ist der Nadelschutzanschlag in Anschlagkontakt mit dem Gehäuseanschlag, sodass das Nadelschutzelement nicht mehr weiter relativ zu dem Gehäuse und in das Gehäuse geschoben werden kann. Dadurch kann sichergestellt werden, dass ein radiales nach aussen Vorspannen oder ein radiales Auslenken der Schulterauflage des Gehäuses verhindert wird. Der Produktbehälter ist mit der distal angeordneten Schulter an der nach innen gerichteten Schulterauflage des Gehäuses abgestützt, um eine axiale relative Bewegung zwischen dem Produktbehälter und dem Gehäuse in die distale Richtung zu verhindern.

In einem weiteren Schritt kann eine Ausgleichsfeder, welche sich an einem proximalen Ende des Produktbehälters abstützt und über die sich der Produktbehälter in und gegen die Ausschüttrichtung elastisch an dem Gehäuse abstützt, in oder an das Gehäuse gehäusefest befestigt werden. In einer alternativen Ausführungsform weist das Gehäuse diese Ausgleichsfeder bereits auf. Die Ausgleichsfeder dient der Sicherstellung des festen Sitzes des Produktbehälters an der Schulterauflage des Gehäuses.

Damit der Patient die Vorrichtung zum Verabreichen der Substanz benutzen kann, muss der Patient vorerst eine an der Vorrichtung lösbar angeordnete Kappe von der Vorrichtung abnehmen. Das Abnehmen der Kappe von der Vorrichtung bewirkt das Entfernen der an der Nadel wegnehmbar angebrachten Nadelschutzhülle. Danach drückt der Patient die Vorrichtung gegen die Haut, wobei das Nadelschutzelement von der distalen Position in die proximale Position relativ zu dem Gehäuse verschoben wird. Bei Überschreitung eines gewissen Schwellenwerts der in die proximale Richtung wirkendenden Kraft, insbesondere der über das Nadelschutzelement wirkenden Kraft auf den Gehäuseanschlag des Gehäuses wird der Gehäuseanschlag radial ausgelenkt, sodass das Nadelschutzelement in eine proximale Position, in welcher eine distale Spitze der Nadel freigeben ist, bewegbar ist. Alternativ kann bei Überschreitung eines gewissen Schwellenwerts der in die proximale Richtung wirkendenden Kraft, insbesondere der über das Nadelschutzelement wirkenden Kraft auf den Gehäuseanschlag des Gehäuses der Nadelschutzelementanschlag radial ausgelenkt werden, sodass das Nadelschutzelement in eine proximale Position, in welcher eine distale Spitze der Nadel freigeben ist, bewegbar ist. Die Nadel wird dabei in die Haut des Patienten gestochen. Das Nadelschutzelement bewegt sich von der distalen Position in die proximale Position, wobei die Antriebseinrichtung gelöst wird, um die Substanz aus dem Produktbehälter zu verabreichen. Die Antriebseinrichtung wird durch eine Ausschüttfeder in eine Ausschüttposition angetrieben, um die Substanz aus dem Produktbehälter zu verabreichen. Am Ende der Verabreichung, kann der Patient die Vorrichtung von der Haut wegnehmen. Durch die Kraft der Nadelschutzfeder wird das Nadelschutzelement von der proximalen Position in die distale Position oder in eine von der distalen Position unterschiedliche distale Position verschoben. Dabei kann das Nadelschutzelement verriegeln, sodass eine erneute Bewegung des Nadelschutzelements in die proximale Position verhindert ist. Der Patient kann die Vorrichtung nicht erneut auslösen.

Die Erfindung wird im Folgenden anhand mehrerer Figuren beschrieben. Die hierbei offenbarten Merkmale bilden je einzeln und in Kombination die Erfindung vorteilhaft weiter. Es zeigen:
- Figur 1: eine perspektivische Längsschnittansicht einer Ausführungsform der erfindungsgemässen Vorrichtung mit einer Längsachse (L) in einem ersten Montageschritt.
- Figur 2: eine perspektivische Längsschnittansicht der Vorrichtung gemäss Figur 1 in einem zweiten Montageschritt.
- Figur 3: eine perspektivische Längsschnittansicht der Vorrichtung gemäss Figur 1 in einem dritten Montageschritt.

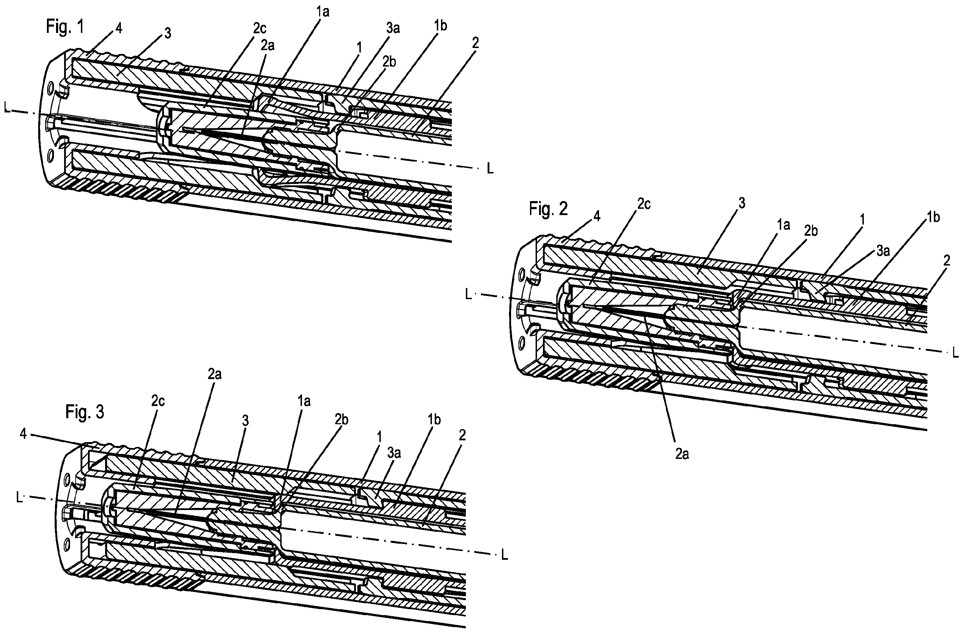

In der Figur 1 ist eine perspektivische Längsschnittansicht einer Ausführungsform der erfindungsgemässen Vorrichtung mit einer Längsachse (L) in einem ersten Montageschritt ersichtlich. Zuerst wird ein Gehäuse (1) mit einer nach innen gerichteten Schulterauflage (1a) zur Aufnahme eines Produktbehälters (2) bereitgestellt. Die nach innen gerichtete Schulterauflage (1a) ist elastisch ausgebildet. Das Gehäuse (1) und die nach innen gerichtete Schulterauflage (1a) sind einteilig oder einstückig ausgebildet. Ferner wird der Produktbehälter (2) mit einer distal angeordneten Schulter (2b) und einer Nadel (2a), welche mit einer entfernbaren Nadelschutzhülse (2c) abgedeckt ist, bereitgestellt.

Wie in der Figur 2 ersichtlich ist, wird in einem zweiten Montageschritt der Produktbehälter (2) in das Gehäuse (1) entlang einer Längsachse (L) in eine distale Richtung verschoben oder eingesetzt. Bei dieser axialen relativen Bewegung zwischen dem Produktbehälters (2) und dem Gehäuse (1) gleitet die nach innen gerichtete Schulterauflage (1a) des Gehäuses (1) über eine äussere Oberfläche der Nadelschutzhülse (2c). Die Schulterauflage (1a) des Gehäuses (1) wird dabei radial nach aussen vorgespannt oder radial ausgelenkt. Wenn die radial nach aussen vorgespannte oder die radial ausgelenkte Schulterauflage (1a) des Gehäuses (1) zwischen einem proximalen Ende der Nadelschutzhülse (2c) und der distal angeordneten Schulter (2b) des Produktbehälters (2) gelangt oder positioniert ist, kann sich die radial nach aussen vorgespannt oder die radial ausgelenkte Schulterauflage (1a) zwischen dem proximalen Ende der Nadelschutzhülse (2c) und der distal angeordneten Schulter (2b) entspannen oder einschnappen. Wenn die Schulterauflage (1a) des Gehäuses (1) zwischen dem proximalen Ende der Nadelschutzhülse (2c) und der distal angeordneten Schulter (2b) des Produktbehälters (2) eingeschnappt ist, ist die Schulterauflage (1a) des Gehäuses (1) entspannt.

Wie in der Figur 3 gezeigt ist, wird in einem dritten Montageschritt das Nadelschutzelement (3) relativ zu dem Gehäuse (1) und in das Gehäuse (1) in die proximale Richtung bis zu einer Stoppposition verschoben. In der Stoppposition der Vorrichtung wird ein erneutes radiales nach aussen Vorspannen oder ein erneutes radiales Auslenken der Schulterauflage (1a) des Gehäuses (1) verhindert. In der Stoppposition der Vorrichtung hält das Nadelschutzelement (3) die Schulterauflage (1a) des Gehäuses (1) in Eingriff mit der Schulter (2b) des Produktbehälters (2). Ein an dem Nadelschutzelement (3) vorgesehener Nadelschutzanschlag (3a) gelangt dabei in Anschlagkontakt mit einem an dem Gehäuse (1) angebrachten Gehäuseanschlag (1b). Der Anschlagkontakt bildet und/oder definiert die Stoppposition der Vorrichtung. Der Gehäuseanschlag (1b) und/oder der Nadelschutzanschlag (3a) können elastisch ausgebildet sein. In der Stoppposition der Vorrichtung ist der Nadelschutzanschlag in Anschlagkontakt mit dem Gehäuseanschlag, sodass das Nadelschutzelement nicht mehr weiter relativ zu dem Gehäuse (1) und in das Gehäuse (1) geschoben werden kann. Der Produktbehälter (2) ist mit der distal angeordneten Schulter (2b) an der nach innen gerichteten Schulterauflage (1a) des Gehäuses (1) abgestützt, um eine axiale relative Bewegung zwischen dem Produktbehälter (2) und dem Gehäuse (1) in die distale Richtung zu verhindern.

In einem weiteren Montageschritt kann eine Ausgleichsfeder, welche sich an einem proximalen Ende des Produktbehälter (2) abstützt und über die sich der Produktbehälter (2) in und gegen die Ausschüttrichtung elastisch an dem Gehäuse (1) abstützt, in oder an das Gehäuse (1) gehäusefest befestigt werden. Die Ausgleichsfeder dient der Sicherstellung des festen Sitzes des Produktbehälters (2) an der Schulterauflage (2b) des Gehäuses (1).

Damit der Patient die Vorrichtung zum Verabreichen der Substanz benutzen kann, muss der Patient zuerst eine an der Vorrichtung lösbar angeordnete Kappe (4) von der Vorrichtung abnehmen. Das Abnehmen der Kappe (4) von der Vorrichtung bewirkt das Entfernen der an der Nadel (2a) wegnehmbar angebrachten Nadelschutzhülle (2c). Danach drückt der Patient die Vorrichtung gegen die Haut, wobei das Nadelschutzelement (3) von der distalen Position in die proximale Position relativ zu dem Gehäuse (1) verschoben wird. Bei Überschreitung eines gewissen Schwellenwerts der in die proximale Richtung wirkendenden Kraft, insbesondere der über das Nadelschutzelement (3) wirkenden Kraft auf den Gehäuseanschlag (1b) des Gehäuses (1) wird der Gehäuseanschlag (1b) radial ausgelenkt, sodass das Nadelschutzelement (3) in eine proximale Position, in welcher eine distale Spitze der Nadel (2a) freigeben ist, bewegt wird. Das Nadelschutzelement (3) bewegt sich von der distalen Position in die proximale Position, wobei eine Antriebseinrichtung gelöst wird, um die Substanz aus dem Produktbehälter (2) zu verabreichen. Die Antriebseinrichtung wird durch eine Ausschüttfeder in eine Ausschüttposition angetrieben, um die Substanz aus dem Produktbehälter (2) zu verabreichen. Am Ende der Verabreichung, kann der Patient die Vorrichtung von der Haut wegnehmen. Durch die Kraft der Nadelschutzfeder wird das Nadelschutzelement (3) von der proximalen Position in die distale Position oder in eine von der distalen Position unterschiedliche distale Position verschoben. Dabei wird das Nadelschutzelement (3) verriegelt, sodass eine erneute Bewegung des Nadelschutzelements (3) in die proximale Position verhindert ist. Der Patient kann die Verrichtung nicht erneut auslösen.

### Bezugszeichen:

1 Gehäuse
1a Schulterauflage
1b Gehäuseanschlag
2 Produktbehälter
2a Nadel
2b Schulter
2c Nadelschutzhülse
3 Nadelschutzelement
3a Nadelschutzanschlag
4 Kappe
L Längsachse

## Patentansprüche

1. Vorrichtung zum Verabreichen einer fluiden Substanz mit einer Längsachse (L) umfassend:
1.1 ein Gehäuse (1);
1.2 ein in dem Gehäuse (1) axial fest aufgenommenen Produktbehälter (2) mit einer Nadel (2a), wobei die Substanz durch die Nadel (2a) verabreichbar ist;
1.3 eine an dem Gehäuse (1) vorgesehene nach innen gerichtete Schulterauflage (1a), an welcher sich der Produktbehälter (2) mit einer distal angeordneten Schulter (2b) abstützt, um eine axiale relative Bewegung zwischen dem Produktbehälter (2) und dem Gehäuse (1) in die distale Richtung zu verhindern;
1.4 ein in dem Gehäuse (1) vorgesehenes und relativ zu dem Gehäuse (1) verschiebbares Nadelschutzelement (3) für die Nadel (2a);
1.5 eine Nadelschutzfeder, welche mit dem Nadelschutzelement (3) gekoppelt ist, wobei das Nadelschutzelement (3) von einer distalen Position, in welcher die distale Spitze der Nadel (2a) von dem Nadelschutzelement (3) umgeben ist und in eine proximale Position, in welcher die distale Spitze der Nadel (2a) von dem Nadelschutzelement (3) freigeben ist, axial bewegbar ist;
1.6 eine in dem Gehäuse (1) bewegbar aufgenommene Antriebseinrichtung, welche durch eine Ausschüttfeder in eine Ausschüttposition getrieben wird, um die Substanz aus dem Produktbehälter (2) zu verabreichen;
1.7 eine an dem Gehäuse (1) vorgesehene Ausgleichsfeder, welche sich an einem proximalen Ende des Produktbehälters (2) abstützt und über die sich der Produktbehälter (2) in und gegen die Ausschüttrichtung elastisch an dem Gehäuse (1) abstützt.

2. Vorrichtung nach Anspruch 1, **dadurch gekennzeichnet, dass** das Nadelschutzelement (3) in der distalen Position ein radiales nach aussen Vorspannen oder radiales Auslenken der Schulterauflage (1a) verhindert.

3. Vorrichtung nach Anspruch 1 oder 2, **dadurch gekennzeichnet, dass** das Nadelschutzelement (3) in der proximalen Position ein radiales nach aussen Vorspannen oder ein radiales Auslenken der Schulterauflage (1a) verhindert.

4. Vorrichtung nach einem der vorhergehenden Ansprüche, **dadurch gekennzeichnet, dass** durch die axiale Bewegung des Nadelschutzelements (3) von der distalen Position in die proximale Position die Antriebseinrichtung gelöst wird, um die Substanz aus dem Produktbehälter (2) zu verabreichen.

5. Vorrichtung nach einem der vorhergehenden Ansprüche, **dadurch gekennzeichnet, dass** die Nadel (2a) des Produktbehälters mit einer entfernbaren Nadelschutzhülse (2c) abgedeckt ist und die nach innen gerichtete Schulterauflage (1a) des Gehäuses (1) zwischen einem proximalen Ende der Nadelschutzhülse (2c) und der distalen Schulter (2b) des Produktbehälters (2) eingeschnappt ist oder eingreift.

6. Verfahren zum Montieren einer Vorrichtung zum Verabreichen einer Substanz nach einem der vorhergehenden Ansprüche mit folgenden Schritten:
- Bereitstellen eines Gehäuses (1) mit einer nach innen gerichteten Schulterauflage (1a) zur Aufnahme eines Produktbehälters (2),
- Bereitstellen eines Produktbehälters (2) mit einer distal angeordneten Schulter (2b) und einer Nadel (1a), welche mit einer entfernbaren Nadelschutzhülse (2c) abgedeckt ist,
- Verschieben oder Einsetzen des Produktbehälters (2) in das Gehäuse (1) entlang einer Längsachse (L) in eine distale Richtung, wobei die nach innen gerichtete Schulterauflage (1a) über eine äussere Oberfläche der Nadelschutzhülse (2c) gleitet und dadurch radial nach aussen vorgespannt wird oder radial ausgelenkt wird,
- Entspannen oder Einschnappen der radial nach aussen vorgespannten oder der radial ausgelenkten Schulterauflage (1a) zwischen einem proximalen Ende der Nadelschutzhülse (2c) und der distal angeordneten Schulter (2b) des Produktbehälters (2), **gekennzeichnet durch**,
- Verschieben des Nadelschutzelements (3) relativ zu und in das Gehäuse (1) in die proximale Richtung bis zu einer Stoppposition der Vorrichtung, bei der ein erneutes radiales nach aussen Vorspannen oder ein erneutes radiales Auslenken der Schulterauflage (1a) verhindert wird.

7. Verfahren nach Anspruch 6, **dadurch gekennzeichnet, dass** in der Stoppposition der Vorrichtung das Nadelschutzelement (3) mit einem Nadelschutzanschlag (3a) an einen Gehäuseanschlag (1b) des Gehäuses (1) in Anschlagkontakt ist.

8. Verfahren nach dem Anspruch 7, **dadurch gekennzeichnet, dass** bei Überschreitung eines gewissen Schwellenwerts der in die proximale Richtung wirkendenden Kraft, insbesondere der über das Nadelschutzelement (3) wirkenden Kraft auf den Gehäuseanschlag (1b) der Gehäuseanschlag (1b) radial ausgelenkt wird, sodass das Nadelschutzelement (3) in eine proximale Position, in welcher eine distale Spitze der Nadel (2a) freigeben ist, bewegbar ist.

9. Verfahren nach Anspruch 8, **dadurch gekennzeichnet, dass** das Nadelschutzelement (3) von der distalen Position in die proximale Position bewegt wird, wobei die Antriebseinrichtung gelöst wird, um die Substanz aus dem Produktbehälter (2) zu verabreichen.

10. Verfahren nach Anspruch 9, **dadurch gekennzeichnet, dass** die Antriebseinrichtung durch eine Ausschüttfeder in eine Ausschüttposition angetrieben wird, um die Substanz aus dem Produktbehälter (2) zu verabreichen.
